(19) Europäisches Patentamt
European Patent Office
Office européen des brevets

(11) **EP 3 799 072 B1**

(12) **EUROPEAN PATENT SPECIFICATION**

(45) Date of publication and mention of the grant of the patent:
**04.05.2022 Bulletin 2022/18**

(21) Application number: **19886727.7**

(22) Date of filing: **22.11.2019**

(51) International Patent Classification (IPC):
**A61K 49/00** (2006.01)   **G16H 50/30** (2018.01)

(52) Cooperative Patent Classification (CPC):
**A61K 49/0004**

(86) International application number:
**PCT/CN2019/120304**

(87) International publication number:
**WO 2020/103941 (28.05.2020 Gazette 2020/22)**

(54) **METHOD FOR CALCULATING TOLERANCE DOSE OF HUMAN BODY TO SUGAR ALCOHOL AND FUNCTIONAL SUGAR**

VERFAHREN ZUR BERECHNUNG DER TOLERANZDOSIS DES MENSCHLICHEN KÖRPERS FÜR ZUCKERALKOHOL UND FUNKTIONELLER ZUCKER

PROCÉDÉ DE CALCUL DE DOSE DE TOLÉRANCE D'UN CORPS HUMAIN À UN ALCOOL DE SUCRE ET UN SUCRE FONCTIONNEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.11.2018 CN 201811422592**

(43) Date of publication of application:
**31.03.2021 Bulletin 2021/13**

(73) Proprietor: **Zhejiang Huakang Pharmaceutical Co., Ltd.**
**Quzhou, Zhejiang 324302 (CN)**

(72) Inventors:
• **ZUO, Qile**
  **Quzhou, Zhejiang 324302 (CN)**
• **HAN, Feifei**
  **Quzhou, Zhejiang 324302 (CN)**
• **ZHU, Xuan**
  **Quzhou, Zhejiang 324302 (CN)**
• **LI, Mian**
  **Santa Clara, California 95054 (US)**
• **ZHANG, Wenyao**
  **Quzhou, Zhejiang 324302 (CN)**
• **SHI, Lihua**
  **Quzhou, Zhejiang 324302 (CN)**

(74) Representative: **Sun, Yiming**
  **HUASUN Patent- und Rechtsanwälte**
  **Friedrichstraße 33**
  **80801 München (DE)**

(56) References cited:
CN-A- 1 651 018    CN-A- 101 356 972
CN-A- 109 493 974    US-A1- 2007 060 639

• S. M. SKOOG ET AL: "Effects of an osmotically active agent on colonic transit.", NEUROGASTROENTEROLOGY AND MOTILITY, vol. 18, no. 4, 1 April 2006 (2006-04-01), pages 300-306, XP055720971, GB ISSN: 1350-1925, DOI: 10.1111/j.1365-2982.2006.00757.x
• SANDROU D K ET AL: "LOW-FAT/CALORIE FOODS: CURRENT STATE AND PERSPECTIVES", CRITICAL REVIEWS IN FOOD SCIENCE AND NUTRITION, TAYLOR & FRANCIS, USA, vol. 40, no. 5, 1 January 2000 (2000-01-01), pages 427-447, XP008031324, ISSN: 1040-8398, DOI: 10.1080/10408690091189211

**Description**

**FIELD OF THE INVENTION**

The present disclosure belongs to the technical field of application of sugar alcohol, and particularly relates to a method for calculating a tolerated dose of a human body to sugar alcohol and functional sugar selected from xylitol, L-arabinose and sorbitol.

**BACKGROUND OF THE INVENTION**

The many benefits of sugar alcohol for a human body are self-evident, but excessive intake also has certain adverse effect on the human body, for example, daily excessive intake of sugar alcohol may cause diarrhea and other situations. Since sugar alcohol enters intestines directly without being decomposed by gastric enzyme, an absorption time is longer than that of glucose in small intestine due to its molecular structure different from sugar, and a large amount of intake in a short time will cause borborygmus and diarrhea. At the same time, some companies have ever declared that the daily intake of xylitol should not exceed 50 g, the daily intake of maltitol should not exceed 100 g, the daily intake of L-arabinose should not exceed 120 g, and the daily intake of sorbitol should not exceed 50 g, etc. However, because no corresponding experimental methods and experimental data prove the above and the dosage requirements are too broad, they have no significant instruction significance for the amount of sugar alcohol added in products.

Skoog S. M. et al., Effects of an osmotically active agent on colonic transit, Neurogastroenterology and Motility (2006) 18, 300-306, disclose a study comparing the effects of sorbitol to glucose, given with or without a meal, on gastrointestinal symptoms, colonic transit, and hydrogen breath excretion in healthy subjects.

**SUMMARY OF THE INVENTION**

The technical problem to be solved by the present disclosure is to provide a method for calculating a tolerated dose of a human body to sugar alcohol and functional sugar selected from xylitol, L-arabinose and sorbitol. By this method, a more accurate range of the tolerated dose of the human body to sugar alcohol and functional sugar can be obtained through animal experiments.

The present invention is realized by providing a method for calculating a tolerated dose of a human body to sugar alcohol and functional sugar selected from xylitol, L-arabinose and sorbitol as defined in the appended claim.

Compared with the prior art, according to the method for calculating the tolerated dose of the human body to sugar alcohol and functional sugar in the present disclosure, a method for calculating a tolerated dose of a human body to sugar alcohol and functional sugar is established by using the most commonly-used xylitol, L-arabinose and sorbitol as the experimental materials and 200 g Wistar rats in animal experiments as the experimental objects in combination with the dose conversion between a human body and an animal in the animal experiments, so as to obtain a more accurate range of the tolerated dose of the human body to sugar alcohol and functional sugar.

**BRIEF DESCRIPTION OF THE DRAWINGS**

FIG. 1 is a statistical diagram of diarrhea situations of rats in experimental groups when a xylitol tolerated dose test is performed for rats according to the present disclosure.

FIG. 2 is a comparison diagram of diarrhea situations of rats in experimental groups on the last day when a xylitol tolerated dose test is performed for rats according to the present disclosure.

FIG. 3 is a statistical diagram of diarrhea situations of rats in experimental groups when an L-arabinose tolerated dose test is performed for rats according to the present disclosure.

FIG. 4 is a comparison diagram of diarrhea situations of rats in experimental groups on the last day when an L-arabinose tolerated dose test is performed for rats according to the present disclosure.

FIG. 5 is a statistical diagram of diarrhea situations of rats in experimental groups when a sorbitol tolerated dose test is performed for rats according to the present disclosure.

FIG. 6 is a comparison diagram of diarrhea situations of rats in experimental groups on the last day when a sorbitol tolerated dose test is performed for rats according to the present disclosure.

## DETAILED DESCRIPTION OF THE EMBODIMENTS

In order to make the technical problems to be solved, technical solutions and advantageous effects of the present disclosure clearer, the present disclosure will be described in detail below with reference to the accompanying drawing and embodiments.

A preferred embodiment of a method for calculating a tolerated dose of a human body to sugar alcohol and functional sugar in the present disclosure includes the following steps 1-5.

At step 1, 200 g rats are selected as experimental animal objects, and the 200 g rats are divided into six experimental groups and one control group, with each group including ten numbered rats; and the rats in the six experimental groups (namely, dose (1) experimental group, dose (2) experimental group, dose (3) experimental group, dose (4) experimental group, dose (5) experimental group and dose (6) experimental group) are gavaged with different doses of xylitol each day. The ten rats in each group are gavaged with the same dose. The ten rats in the control group are gavaged with the same dose of sucrose or normal saline each day; enough drinking water and common feed are provided to all the experimental rats each day; whether there is fecal matter at the anus of each rat in each group is checked every night, wherein the existence of the fecal matter indicates that the rat has diarrhea, and statistics are made on the quantity and numbers of rats with diarrhea.

At step 2, the step 1 is continued for 14 days.

At step 3, by combining the diarrhea situations of rats on the last day (day 14) with results of intestinal segment slice maps and diamine oxidase contents of the rats in each group, a maximum dose group in which all the rats do not have diarrhea is used to obtain a maximum tolerated dose N of the rats in the group to xylitol.

The results of the intestinal segment slices and the diamine oxidase of the rats prove that the rats with diarrhea found through observation are subjected to gastrointestinal damage due to their doses exceeding a normal tolerated dose, and the intestinal segment slice maps and the diamine oxidase contents of the rats still having diarrhea on the last day (day 14) are significantly different from those of the rats in the control group and the rats without diarrhea. Therefore, the maximum dose group in which all the rats in this group do not have diarrhea on the last day can be taken as the maximum tolerated dose.

At step 4, the maximum tolerated dose N of the 200 g rats to xylitol is converted according to the following conversion formula of drug doses of experimental animal and human body to obtain a tolerated dose R of the human body to xylitol:

$$\text{70 kg-human-body dose R} = \text{maximum tolerated dose N of 200 g rat} \times K$$

wherein K is a conversion factor between a 70 kg human body and a 200 g rat, which is 56.0. The maximum tolerated dose N of the rat to xylitol and the tolerated dose R of the human body to the xylitol are both in the unit of g/d; or,

at step 5, the xylitol in the step 1 is replaced with L-arabinose, and the steps 1-4 above are repeated to obtain a tolerated dose of the 70 kg human body to L-arabinose; or,

the xylitol in the step 1 is replaced with sorbitol, and the steps 1-4 are repeated to obtain a tolerated dose of the 70 kg human body to sorbitol.

In the specific implementation, the maximum tolerated dose of the human body to a certain sugar alcohol or functional sugar recorded in the existing data is used as a reference value firstly. Taking xylitol as an example, the existing companies have declared that the daily intake of xylitol should not exceed 50g. 50 g of xylitol is set as the reference value, and fluctuation is performed based on the reference value to obtain an estimated value of the experimental range. According to the conversion formula of drug doses of experimental animal and human body, the daily experimental dose of 200 g rats to xylitol is obtained through conversion, the group experiment is performed according to the daily experimental dose and statistics are made on the experimental results each day. The experiment is continued for 14 days. Considering the experimental results and verification results comprehensively, the maximum tolerated dose N of 200 g rats to xylitol is obtained. Then, according to the maximum tolerated dose N, the most appropriate maximum tolerated dose R of the human body to xylitol is obtained by conversion based on the conversion formula of drug doses of experimental animal and human body.

Similarly, the maximum tolerated doses of the human body to L-arabinose and sorbitol can be obtained.

Specifically, 50 g of xylitol was set as the reference value, and estimated values of the experimental range of the human body to xylitol were set to 20 g/d, 30 g/d, 40 g/d, 50 g/d, 60 g/d and 70 g/d (six groups in total) according to the reference value. According to the conversion formula of drug doses of experimental animal and human body, the daily experimental dose of xylitol of 200 g rats was respectively obtained as 0.36 g/d, 0.54 g/d, 0.71 g/d, 0.89 g/d, 1.07 g/d and 1.25 g/d (six groups in total), and then experiments were performed respectively.

Similarly, estimated values of the human body to L-arabinose were respectively set to 70 g/d, 80 g/d, 90 g/d, 100 g/d, 110 g/d and 120g/d (six groups in total) as the experimental range. According to the conversion formula of drug doses of experimental animal and human body, the daily experimental dose of L-arabinose of 200 g rats was respectively obtained as 1.25 g/d, 1.43 g/d, 1.61 g/d, 1.79 g/d, 1.96 g/d and 2.14 g/d (six groups in total), and then experiments were performed respectively.

Similarly, estimated values of the human body to sorbitol were respectively set to 5 g/d, 10 g/d, 15 g/d, 20 g/d, 25 g/d and 30 g/d (six groups in total) as the experimental range. According to the conversion formula of drug doses of experimental animal and human body, the daily experimental dose of sorbitol of 200 g rats was respectively obtained as 0.09 g/d, 0.18 g/d, 0.27 g/d, 0.36 g/d, 0.45 g/d and 0.54 g/d (six groups in total), and then experiments were performed respectively.

The experimental dose of 200 g rats in the control group was 0.5 g/d sucrose solution.

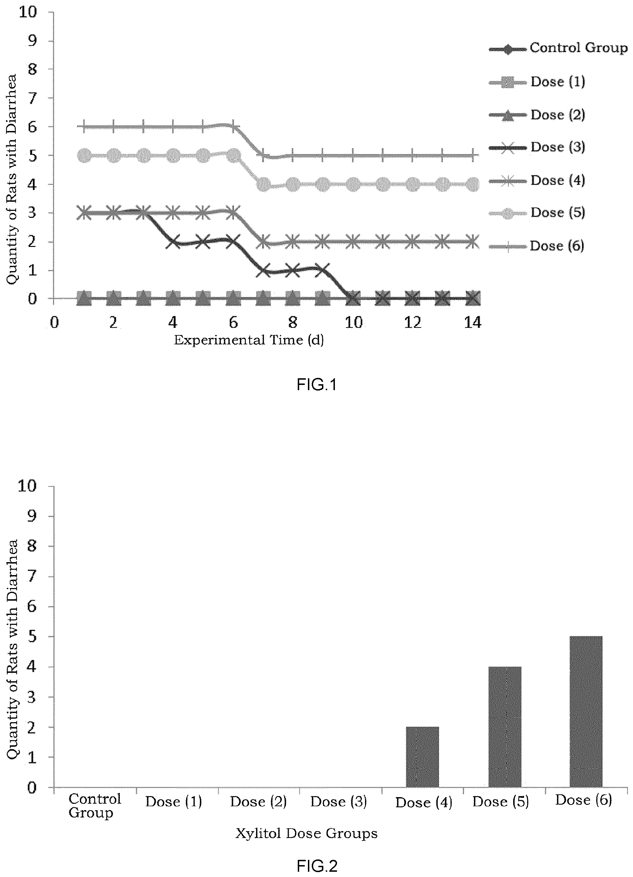

FIG. 1 to FIG. 6 are the statistics of diarrhea situations of 200 g rats for sugar alcohol and functional sugar during an experimental period. For each sugar alcohol or functional sugar, the experimental rats theoretically have a maximum sugar alcohol or functional sugar tolerated dose threshold, so the following three situations may occur during feeding:

(1) no diarrhea occurred during the experimental period;

(2) the diarrhea occurred at the beginning, then improved until the diarrhea disappeared (rats had tolerance adaptivity);

(3) diarrhea existed throughout the experimental period.

Therefore, the maximum tolerated dose is divided according to the above: situation (1) and situation (2) are regarded as within the tolerable range, situation (3) is regarded as beyond the tolerable range, and the maximum dose of situation (2) is considered to be the maximum tolerated dose of the sugar alcohol or functional sugar.

Referring to FIG. 1 and FIG. 2, when the xylitol tolerated dose experiment was performed with the rats, the maximum sugar tolerance dose was dose (3), that is, the dose for gavaging the rats was 0.71 g/d, and the maximum tolerated dose of the human body to xylitol was calculated as 0.71*56.0=40 g/d according to the conversion formula of drug doses of experimental animal and human body. The maximum tolerated dose 40 g of the human body to xylitol obtained by the method of the present disclosure is more specific, more accurate and more well-grounded than the currently-known daily xylitol intake amount of the human body which is preferably no more than 50 g.

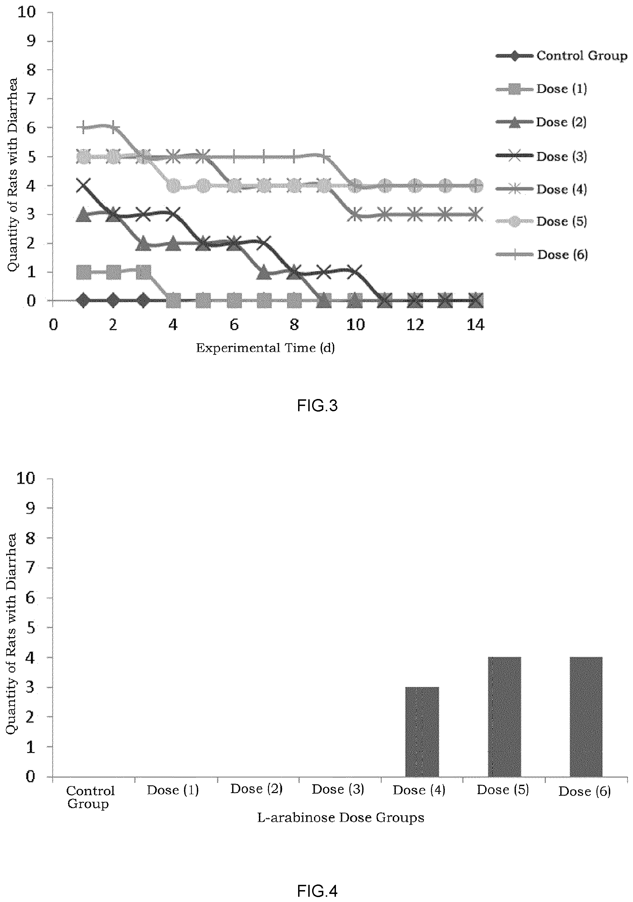

Referring to FIG. 3 and FIG. 4, when the L-arabinose tolerated dose experiment was performed with the rats, the maximum sugar tolerance dose was dose (3), that is, the dose for gavaging the rats was 1.61 g/d, and the maximum tolerated dose of the human body to xylitol was calculated as 1.61*56.0=90 g/d according to the conversion formula of drug dose of experimental animal and human body.

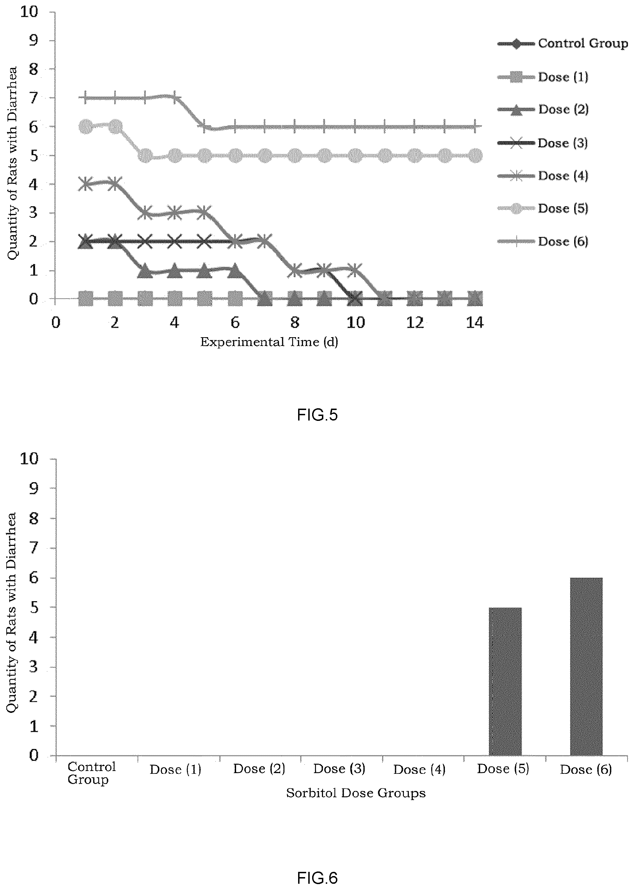

Referring to FIG. 5 and FIG. 6, when the sorbitol tolerated dose experiment was performed with the rats, the maximum sugar tolerance dose was dose (4), that is, the dose for gavaging the rats was 0.36 g/d, and the maximum tolerated dose of the human body to xylitol was calculated as 0.36*56.0=20 g/d according to the conversion formula of drug doses of experimental animal and human body.

## Claims

1. A method for calculating a tolerated dose of a human body to sugar alcohol and functional sugar selected from xylitol, L-arabinose and sorbitol, comprising the following steps:

at step 1, selecting 200 g rats as experimental animal objects, and dividing the 200 g rats into six experimental groups and one control group, with each including ten numbered rats; gavaging different doses of xylitol, L-arabinose or sorbitol to the rats in the six experimental groups each day, wherein the ten rats in each group are gavaged with the same dose, and gavaging the same dose of sucrose or normal saline to the ten rats in the control group each day; providing enough drinking water and common feed to all the experimental rats each day, checking whether there is fecal matter at the anus of each rat in each group every night, wherein the existence of the fecal matter indicates that the rat has diarrhea, and making statistics on the quantity and numbers of rats with diarrhea;

at step 2, continuing the step 1 for 14 days;

at step 3, by combining the diarrhea situations of rats on the last day (day 14) with results of intestinal segment slice maps and diamine oxidase contents of the rats in each group, using a maximum dose group in which all the rats do not have diarrhea to obtain a maximum tolerated dose N of the rats in the group to xylitol, L-arabinose or sorbitol;

at step 4, converting the maximum tolerated dose N of the 200 g rats to xylitol, L-arabinose or sorbitol according to the following conversion formula of drug doses of experimental animal and human body to obtain a tolerated dose R of the human body to xylitol, L-arabinose or sorbitol:

$$70 \text{ kg-human-body dose } R = \text{maximum tolerated dose } N \text{ of } 200 \text{ g rat} \times K$$

wherein K is a conversion factor between a 70 kg human body and a 200 g rat, which is 56.0; the maximum tolerated dose N of the rat to xylitol, L-arabinose or sorbitol and the tolerated dose R of the human body to the xylitol, L-arabinose or sorbitol are both in the unit of g/d.

## Patentansprüche

1. Verfahren zum Berechnen einer tolerierten Dosis eines menschlichen Körpers gegenüber Zuckeralkohol und funktionellem Zucker, ausgewählt aus Xylit, L-Arabinose und Sorbitol, umfassend die folgenden Schritte:

in Schritt 1, Auswahl von 200 g schweren Ratten als Versuchstiere und Aufteilung der 200 g schweren Ratten in sechs Versuchsgruppen und eine Kontrollgruppe, wobei jede Gruppe zehn nummerierte Ratten enthält; tägliche Verabreichung unterschiedlicher Dosen von Xylit, L-Arabinose oder Sorbitol an die Ratten in den sechs Versuchsgruppen, wobei die zehn Ratten in jeder Gruppe mit der gleichen Dosis verabreicht werden, und tägliche Verabreichung der gleichen Dosis von Saccharose oder normaler Kochsalzlösung an die zehn Ratten in der Kontrollgruppe; Bereitstellung einer ausreichenden Menge an Trinkwasser und gemeinsamem Futter für alle Versuchsratten jeden Tag, Überprüfung, ob sich am Anus jeder Ratte in jeder Gruppe jede Nacht Fäkalien befinden, wobei das Vorhandensein der Fäkalien anzeigt, dass die Ratte Durchfall hat, und Erstellung von Statistiken über die Menge und Anzahl der Ratten mit Durchfall;

in Schritt 2 Fortsetzung des Schritts 1 über 14 Tage;

in Schritt 3, durch Kombinieren der Durchfall-Situationen der Ratten am letzten Tag (Tag 14) mit Ergebnissen von Darmsegment-Schnittkarten und Diamin-Oxidase-Gehalten der Ratten in jeder Gruppe, unter Verwendung einer Gruppe mit maximaler Dosis, in der alle Ratten keinen Durchfall haben, um eine maximal tolerierte Dosis N der Ratten in der Gruppe für Xylit, L-Arabinose oder Sorbitol zu erhalten;

in Schritt 4 Umrechnung der maximal verträglichen Dosis N der 200 g schweren Ratten in Xylit, L-Arabinose oder Sorbitol gemäß der folgenden Umrechnungsformel der Arzneimitteldosen von Versuchstier und menschlichem Körper, um eine verträgliche Dosis R für den menschlichen Körper auf Xylit, L-Arabinose oder Sorbitol zu erhalten:

$$70 \text{ kg-Mensch-Körper-Dosis } R = \text{maximal tolerierte Dosis } N \text{ von } 200 \text{ g Ratte} * K$$

wobei K ein Umrechnungsfaktor zwischen einem 70 kg schweren menschlichen Körper und einer 200 g schweren Ratte ist, der 56,0 beträgt; die maximal tolerierte Dosis N der Ratte für Xylit, L-Arabinose oder Sorbitol und die tolerierte Dosis R des menschlichen Körpers für Xylit, L-Arabinose oder Sorbitol liegen beide in der Einheit g/d vor.

## Revendications

1. Procédé de calcul d'une dose tolérée d'un corps humain à l'alcool sucré et au sucre fonctionnel choisi parmi le xylitol, le L-arabinose et le sorbitol, comprenant les étapes suivantes :

à l'étape 1, sélectionner 200 g de rats comme objets animaux expérimentaux et diviser les 200 g de rats en six groupes expérimentaux et un groupe témoin, avec chacun comprenant dix rats dénombrés ; gaver différentes doses de xylitol, de L-arabinose ou de sorbitol aux rats dans les six groupes expérimentaux chaque jour, les dix rats dans chaque groupe étant gavés avec la même dose, et gaver la même dose de saccharose ou de solution saline normale aux dix rats du groupe témoin chaque jour ; fournir suffisamment d'eau de boisson et d'aliments communs à tous les rats expérimentaux chaque jour, vérifier s'il y a de la matière fécale à l'anus de chaque rat dans chaque groupe chaque nuit, l'existence de matière fécale indiquant que le rat a la diarrhée, et faire des statistiques sur la quantité et les nombres de rats avec la diarrhée ;

à l'étape 2, continuer l'étape 1 durant 14 jours ;

à l'étape 3, en combinant les situations de diarrhée des rats au dernier jour (jour 14) avec les résultats des cartes de tranche de segment intestinal et les teneurs en diamine oxydase des rats dans chaque groupe, utiliser un groupe de dose maximum dans lequel tous les rats n'ont pas la diarrhée pour obtenir une dose maximum tolérée N des rats dans le groupe au xylitol, au L-arabinose ou au sorbitol ;

à l'étape 4, convertir la dose tolérée maximum N des 200 g de rats au xylitol, au L-arabinose ou au sorbitol selon la formule de conversion suivante de doses de médicament d'animal expérimental et de corps humain pour obtenir une dose tolérée R du corps humain au xylitol, L-arabinose ou sorbitol ;

$$\text{dose R de 70 kg de corps humain} = \text{dose maximum tolérée N de 200 g de rat} \times K$$

K étant un facteur de conversion entre un corps humain de 70 kg et un rat de 200 g, qui est de 56,0 ; la dose tolérée maximum N du rat au xylitol, L-arabinose ou sorbitol et la dose tolérée R du corps humain au xylitol, L-arabinose ou sorbitol sont toutes les deux dans l'unité de g/j.

FIG.1

FIG.2

FIG.3

FIG.4

FIG.5

FIG.6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SKOOG S. M. et al.** Effects of an osmotically active agent on colonic transit. *Neurogastroenterology and Motility,* 2006, vol. 18, 300-306